# NEUE EUROPÄISCHE PATENTSCHRIFT

(11) **EP 1 904 000 B3**
(45) Veröffentlichungstag dieser Patentschrift: **13.02.2013**
(45) Hinweis auf die Patenterteilung: 20.10.2010
(21) Anmeldenummer: 06742321.0
(22) Anmeldetag: 09.05.2006
(51) Int. Cl.: A61F 5/00

(54) **BANDAGE, INSBESONDERE ALS TRAGELEMENT EINER ORTHESE**
BANDAGE, ESPECIALLY AS A SUPPORTING ELEMENT OF AN ORTHOSIS
BANDAGE SERVANT EN PARTICULIER D'ELEMENT DE SUPPORT POUR UNE ORTHESE

(30) Priorität: 05.07.2005 DE 102005031867
(43) Veröffentlichungstag der Anmeldung: 02.04.2008
(73) Patentinhaber: Otto Bock HealthCare GmbH, 37115 Duderstadt (DE)
(72) Erfinder: VOLLBRECHT, Matthias, 37412 Herzberg (DE)
(74) Vertreter: Lins, Edgar
(86) Internationale Anmeldenummer: PCT/DE2006/000812
(87) Internationale Veröffentlichungsnummer: WO 2007/003148

(56) Entgegenhaltungen:
- WO-A-2004/065677
- US-A- 4 556 055
- US-A- 5 363 863
- US-A- 5 823 984

## Beschreibung

Die Erfindung betrifft eine Bandage, bestehend aus mehreren Teilen, die zugfestmiteinanderverbindbar sind.

Bandagen der hier erwähnten Art dienen zur Umschließung eines Körperbereichs, insbesondere eines Rumpfes eines Patienten. Derartige Bandagen können mit einer Stützeinrichtung versehen sein, um so beispielsweise eine Rumpforthese zur Stützung und Entlastung der Lendenwirbelsäule einzusetzen. Es ist bekannt, dass dabei ganz unterschiedliche Funktionen der Orthese erforderlich werden können. So kann es geboten sein, den Lordosenbereich der Wirbelsäule vollständig zu entlasten, indem er durch die Stützeinrichtung überbrückt wird. Hierbei wird eine weitgehende Immobilisierung der Wirbelsäule bewirkt.

Ferner ist es bekannt, den Lumbalbereich bzw. lumbosakralen Bereich der Wirbelsäule während einer eingeschränkten Beweglichkeit zu stützen. In einer weitergehenden Rehabilitationsphase kann es ggf. nur noch erforderlich sein, eine gewisse Stützwirkung mittels einer Bandage oder einer geringfügig verstärkten Bandage auszuüben.

Durch DE 202 04 747 U1 ist eine Rumpforthese der eingangs erwähnten Art bekannt, die auf eine vielseitige Einsetzbarkeit für die verschiedenen Anwendungsfälle und auf die Anpassung an unterschiedliche Patienten ausgerichtet ist. Neben einem Aufbau der Bandage aus zwei überlappenden Teilbandagen, die eine Anpassung der Bandagenhöhe an den jeweiligen Patienten ermöglichen soll, sind für die Bandage unterschiedliche Stützeinrichtungen vorgesehen. Außer in vorgesehene Taschen einschiebbaren Stützstäben können an der Bandagenanordnung unterschiedliche Stützeinrichtungen in Form eines Rückenstützrahmens zur Überbrückung des Lordosenbereichs (Entlordosierung) oder eine Rückengliederpelotte zur Stabilisierung des bewegbaren Lordosenbereichs befestigt werden. Ggf, kann diese Wirbelsäulenorthese durch eine schalenförmige Bauchpelotte ergänzt werden. Die unterschiedlichen Stützeinrichtungen können dabei mittels Klettbandverschlüssen an der Bandage befestigt und somit in einfacher Weise ausgewechselt werden.

Die bekannten Bandagen werden in unterschiedlichen Längen hergestellt, um eine Anpassung an unterschiedliche Umfangsmaße des umschlungenen Körperbereichs vornehmen zu können, Dabei reicht eine grobe Abstufung der Längen aus, weil die Bandage an ihren Enden mehr oder weniger stark überlappen kann. Eine einzige, universell verwendete Länge würde jedoch zu sehr hohen Überlappungsmaßen für einen geringen Umfang des jeweiligen Körperbereichs führen, wodurch der Sitz der Bandage verschlechtert und unbequem wird. Die Herstellung von Bandagen unterschiedlicher Länge und deren Lagerhaltung erfordert somit einen gewissen Aufwand.

US 5,823,984 offenbart eine Bandage, die aus mehreren gleichen Bandageabschnitten besteht, die über Klettverschlüsse miteinander verbindbar sind. Hierzu ist die Oberfläche der Bandagenabschnitte mit einem Schlaufengewebe einer Klettverbindung versehen. An einem Ende der Bandagenabschnitte befinden sich jeweils über den Bandagenabschnitten ragende Befestigungsstreifen, die auf ihrer Unterseite mit hakenförmigem Gewebe für die Klettverbindung ausgestattet sind. Durch die Aneinanderreihung mehrerer Bandagenabschnitte und durch eine Variation der Überlappung dieser Bandagenabschnitte können den Körperumschließende Bandgagen gebildet werden, die zum Andrücken eines Kühlelements oder eines Wärmeelements verwendet werden. Das Kühl- oder Wärmeelement kann dabei in eine Tasche eingebracht werden, die eine Wandung mit hakenförmigen Klettverschlusselementen aufweist und so mit der Oberfläche eines Beliebigen der Bandagenabschnitte verbunden werden kann. Die Ausbildung einer Orthese ist mit der bekannten Bandage nicht vorgesehen,

Der vorliegenden Erfindung liegt die Aufgabe zugrunde, eine Bandage der eingangs erwähnten Art so auszubilden, dass sie für unterschiedliche benötigte Längen weniger aufwändig hergestellt und auf Lager gehalten werden kann und für die Ausbildung einer Orthese, insbesondere Rumpforthese, ausgebildet werden kann.

Diese Aufgabe wird mit einer Bandage der eingangs erwähnten Art dadurch gelöst, dass wenigstens ein Mittelstück und zwei zum Schließen der Bandage miteinander verbindbare Endstücke vorgesehen sind und dass zur Herstellung einer zugfesten Verbindung zwischen Teilen der Bandage ein Teil ein flaches Ende mit beidseitig angebrachten Klettverschlusselementen und der zu verbindende andere Teil mit einem das flache Ende beidseitig übergreifenden maulartigen Ende mit Klettverschlussgegenelementen auf den an dem flachen Ende anliegenden Innenseiten versehen ist und dass sich die zugfeste Verbindung über die gesamte Breite der Bandage erstreckt.

Die zugfeste Verbindung wird zwischen den Teilen mit Klettverschlüssen gebildet, die sich dabei über die gesamte Breite der Bandage erstrecken, sodass eine große Verschlussfläche zur Verfügung steht. Auf diese Weise gelingt es, eine zugfeste Verbindung herzustellen, die bei einer Bandage, die eine Körperregion, beispielsweise den Rumpf, unter einer gewissen Spannung umschließen soll, unbedingt benötigt wird.

Erfindungsgemäß ist zur Sicherstellung einer hohen Zugfestigkeit der Verbindung zwischen den Teilen der Bandagen vorgesehen, dass ein Teil der Bandage ein flaches Ende mit beidseitig angebrachten Klettverschlusselementen und der zu verbindende andere Teil ein das flache Ende beidseitig übergreifendes maulartiges Ende mit Klettverschlussgegenelementen auf den an dem flachen Ende anliegenden Innenseiten aufweist. Dadurch wird die wirksame Verschlussfläche für den Klettverschluss zwischen den Teilen verdoppelt, ohne dass eine größere Dimensionierung der Klettverschlussstreifen in Längsrichtung der Bandage erforderlich wäre. Darüber hinaus wird durch diese Ausbildung dem Auftreten eines Drehmoments in Richtung des Lösens der Klettverschlüsse entgegengewirkt, da die Zugkraft auf beiden Seiten der miteinander verbundenen Enden der Teile der Bandage wirksam wird.

Die erfindungsgemäße Bandage lässt sich vorteilhaft als Träger für eine Stützeinrichtung ausbilden.

Die erfindungsgemüße Bandage lässt sich in mehreren Längen ausbilden. Eine erste, geringste Länge entsteht dadurch, dass die Endstücke unmittelbar an das Mittelstück angesetzt werden. Größere Längen entstehen dadurch, dass zwischen dem Mittelstück und den beiden Endstücken jewels gleich lange Zwischenstücks eingesetzt werden, die kürzer oder länger ausgebildet sein konnen.

Im Rahmen der Erfindung ist es auch möglich, mehrere Zwischenstücke aneinander anschließen zu lassen, um größere Längen zu realisieren. Bevorzugt ist jedoch die Ausbildung mit unterschiedlich langen Zwischenstücken, um die Anzahl der Klettverbindungen nicht zu groß werden zu lassen.

Die Erfindung soll im Folgenden anhand eines in der Zeichnung dargestellten Ausführungsbeispiels näher erläutert werden. Es zeigen:
- Figur 1: eine perspektivische Darstellung einer flachliegenden mehr-teiligen (nicht zusammengefügten) Bandage;
- Figur 2: eine Darstellung gemäß Figur 1 einer für eine geelgnete Länge zusammengefügten Bandage;
- Figur 3: die Darstellung gemäß Figur 2 mit eineraufgebrachten Stützeirichtung mit einem durch die Stützeihrichtung geführ- ten Spanngurt.

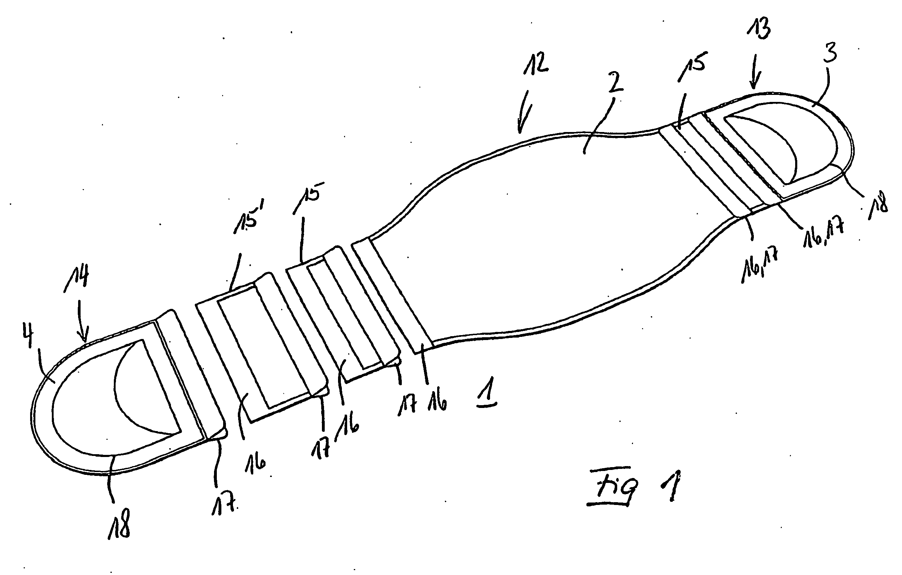

Das in dar Zeichnung dargestellte Ausführungsbelsplel einer Bandage 1 ist mehrstückig ausgebildet, wobei ein Mittelstück 12 den breiteren mittleren Bereich 2 und Endstücke 13, 14 die Endbereiche 3, 4 bilden. Zwischen dem Mittelstück 12 und den Endstücken 13, 14 ist ein Zwischenstück 15, 15' einsetzbar, wobei Zwischenstücke 15, 15' unterschiedilcher Länge zur Verfügung stehen, um unterschledfliche lange Bandagen 1 zu ermöglichen. In Figur 1 ist zum rechten Endstück 13 ein eingesetztes und mit dem Mittelstück 2 und dem Endstück 13 verbundenes Zwischenstück 15 dargestellt, während auf der linken Seite die zur Verbindung möglichen Zwischenstücke 15, 15' unterschirdlicher Länge einzeln gezeigt sind.

Wenn das zum Bauchumfang des Patienten 10 passende Zwischenstück 15, 15' geeigneter Länge ausgewähit ist, wird es mit dem zugehörigen Endstück 13, 14 und dem Mittelstück 2 zugfest verbunden.

Im dargestellten Ausführungsbeispiel erfolgt die zugfeste Verbindung zwischen dem Zwischenstück 15, 15' und dem Endstück 14 dadurch, dass das Zwischenstück 15, 15' ein zum Endstück 13, 14 zeigendes flaches Ende 16 aufweist, das beidseitig mit Klettverschlussellmenten versehen ist. Die Endstücke 13, 14 weisen zu den Zwischenstücken 15, 15' zeigende maulartige Enden 17 auf, die das flache Ende 16 des Zwischenstückes 15, 15' beidseitig übergreifen und mit entsprechenden Klettverschlussgegenelementen versehen sind, sodass beidseitig des flachen Endes 16 eine Klettverschlussverbindung mit den Endstücken 13, 14 hergestellt wird.

In entsprechender Weise ist das Mittelstück 12 mit flerchen Enden 16 ausgebidet, die mit zum Mittelstück 12 zeigenden mautartigen Enden 17 des Zwischenstücks 15, 15' zur Herslellung einer zugfesten Klettverbindung 16, 17 zusammenwirken.

Figur 1 lässt noch erkennen, dass die Endstükke 13, 14 an Ihrer (in der Darstellung oberen) Außenseite eine aufgesetzte Tasche 18 aufweisen, in die der Patient 10 mit seinen Händen eingreifen kann, um die Bandage 1 um seinen Rumpf herum mit einem gewissen Zug zu befestigen.

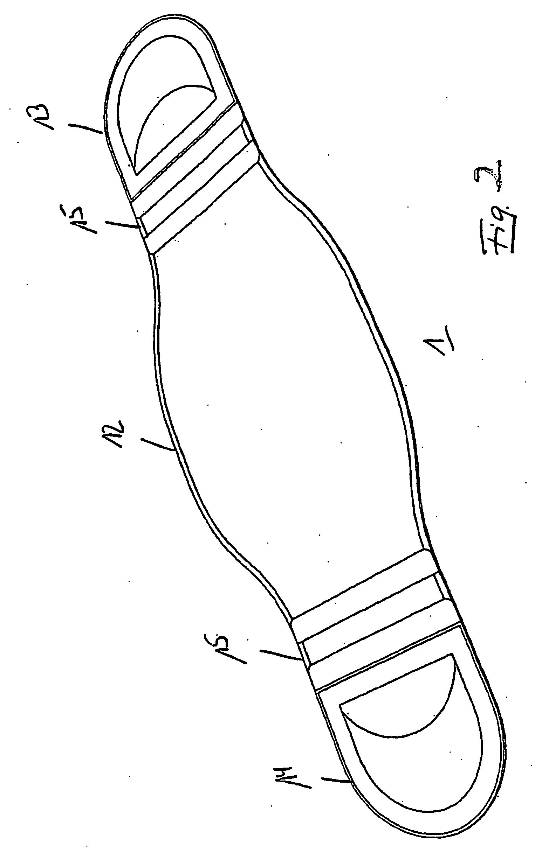

Figur 2 zeigt die nach Auswahl des geeigneten Zwischenstücks15, 15' zusammengestellte gebrauchsfertige Bandage 1.

Selbstverständlich kann die Bandage 1 biel geringen Körperumfängen auch ohne ein Zwischenstück 15, 15' zusammengestelltwerden, Indem die Endstücke 13, 14 unmittelbar an dem Mittelstück 12 befestigt werden

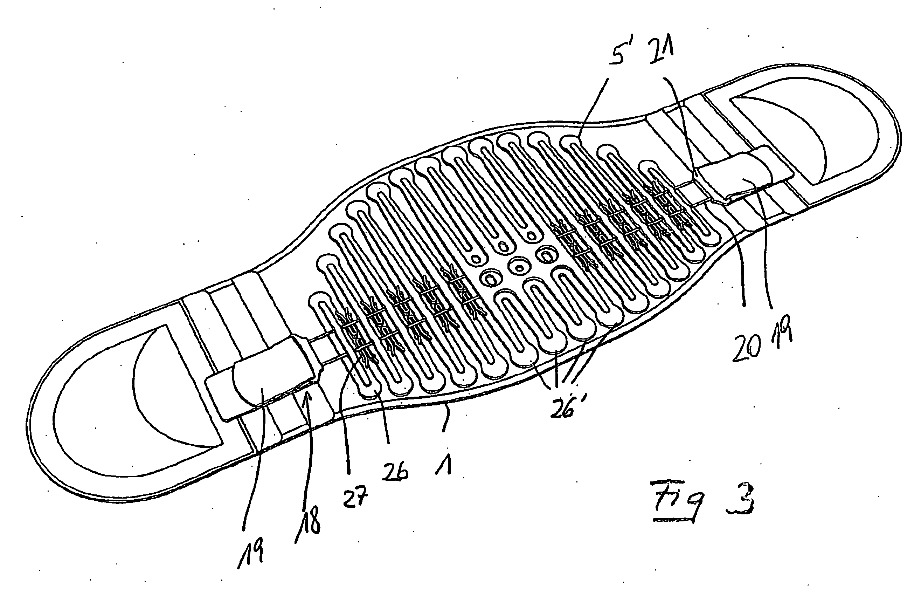

Gemäß Figur 3 kann auf die Bandage 1. eine Slützeinrichtung 5' aufgebracht werden, die aus nebeneinander angeordneten Fingerstäben 26 besteht, die durch einstückig mit den Fingerstäben ausgebildete Federelemente 27 miteinander verbunden sind. Die Federelemente 27 sind U-förmig ausgebildet

Die erfindungsgemäß Bandage ist somit ohne Stützeirichtung 5', aber auch mit der Stützeinrichtung 5' verwendbar.

## Patentansprüche

1. Bandage aus mehreren Teilen, die zugfest miteinander verbindbar sind, mit wenigstens einem Mittelstück (12) und zwei zum Schließen der Bandage miteinander verbindbare Endstücken (13, 14) und zur Herstellung einer zugfesten Verbindung zwischen Teilen der Bandage ein Teil ein flaches Ende (16) mit beidseitig angebrachten Klettverschlusselementen aufweist und **dadurch gekennzeichnet ist, dass** der zu verbindende andere Teil mit einem das flache Ende (16) beidseitig übergreifenden maulartigen Ende (17) mit Klettverschlussgegenelementen auf den an dem flachen Ende (16) anliegenden Innenseiten versehen ist und dass sich die zugfeste Verbindung über die gesamte Breite der Bandage erstreckt.

2. Bandage nach Anspruch 1, **dadurch gekennzeichnet, dass** das Mittelstück (12) einen breiteren mittleren Bereich (2) bildet.

3. Bandage nach Anspruch 1 oder 2, **dadurch gekennzeichnet, dass** zwischen dem Mittelstück (12) und einem Endstück (13, 14) wenigstens ein Zwischenstück (15, 15') zur Verstellung der Länge der Bandage einsetzbar ist.

4. Bandage nach Anspruch 3, **dadurch gekennzeichnet, dass** sie mehrere zwischen Mittelstück (12) und Endstück (13, 14) einsetzbare Zwischenstücke (15, 15') aufweist.

5. Bandage nach Anspruch 3 oder 4, **dadurch gekennzeichnet, dass** unterschiedlich lange Zwischenstücke (15, 15') zum Einsatz zwischen Mittelstück (12) und Endstück (13, 14) vorgesehen sind.

## Claims

1. Bandage consisting of several parts which can be connected strongly with one another, with at least one middle piece (12), and two end pieces (13, 14) that can be connected together to close the bandage, and, in order to produce a strong connection between parts of the bandage, one part has a flat end (16) with Velcro fastener elements attached on both sides, and is **characterized in that** the other part that is to be connected is provided with a mouth-like end (17) which goes over both sides of the flat end (16) and which has Velcro fastener counter-elements on the inner surfaces that lie against the flat end (16) and **in that** the strong connection extends across the entire width of the bandage.

2. Bandage according to Claim 1, **characterized in that** the middle piece (12) forms a wider middle area (2).

3. Bandage according to Claim 1 or 2, **characterized in that**, between the middle piece (12) and an end piece (13, 14), it is possible to insert at least one intermediate piece (15, 15') in order to adjust the length of the bandage.

4. Bandage according to Claim 3, **characterized in that** it has several intermediate pieces (15, 15') insertable between middle piece (12) and end piece (13, 14).

5. Bandage according to Claim 3 or 4, **characterized in that** intermediate pieces (15, 15') of different lengths are provided for use between middle piece (12) and end piece (13, 14).

## Revendications

1. Bandage constitué de plusieurs parties qui peuvent être reliées les unes aux autres de façon résistante à la traction, comprenant au moins une pièce centrale (12) et deux pièces extrêmes (13, 14) pouvant être reliées ensemble pour fermer le bandage, et une partie présente, pour l'obtention d'une liaison résistante à la traction entre parties du bandage, une extrémité plate (16) ayant des éléments de fermeture auto agrippante placés des deux côtés, et **caractérisé en ce que** l'autre partie à relier est munie d'une extrémité en mâchoire (17) recouvrant des deux côtés l'extrémité plate (16) et comprenant des éléments de fermeture auto agrippante sur les faces intérieures adjacentes à l'extrémité plate (16), et **en ce que** la liaison résistante à la traction s'étend sur toute la largeur du bandage.

2. Bandage selon la revendication 1, **caractérisé en ce que** la pièce centrale (12) forme une région (2) centrale relativement large.

3. Bandage selon la revendication 1 ou 2, **caractérisé en ce qu'**au moins une pièce intermédiaire (15, 15') peut être insérée entre la pièce centrale (12) et une pièce extrême (13, 14) pour régler la longueur du bandage.

4. Bandage selon la revendication 3, **caractérisé en ce qu'**il présente plusieurs pièces intermédiaires (15, 15') pouvant être insérées entre pièce centrale (12) et pièce extrême (13, 14).

5. Bandage selon la revendication 3 ou 4, **caractérisé en ce que** des pièces intermédiaires (15, 15') de différentes longueurs sont prévues pour être insérées entre pièce centrale (12) et pièce extrême (13, 14).
